# EUROPÄISCHE PATENTANMELDUNG

(11) **EP 4 811 371 A1**
(43) Veröffentlichungstag der Anmeldung: **23.09.2026**
(21) Anmeldenummer: 26165316.6
(22) Anmeldetag: 17.03.2026
(51) Int. Cl.: G16H 20/30, G16H 10/60, G16H 40/20, G16H 50/20, G16H 70/20

(54) **VERFAHREN ZUR PLANUNG UND/ODER ZUR MODIFIKATION UND/ODER ZUR STEUERUNG UND/ODER ZUR BEREITSTELLUNG VON DATEN UND/ODER INFORMATION, DIE EINEN PFLEGEPROZESS EINES PATIENTEN BETREFFEN**

(30) Priorität: 18.03.2025 DE 102025110471
(71) Anmelder: ePA-CC GmbH, 65203 Wiesbaden (DE)
(72) Erfinder: Fiebig, Madlen, 64289 Darmstadt (DE); Hunstein, Dirk, 65199 Wiesbaden (DE); Sippel, Birgit, 88048 Friedrichshafen (DE)
(74) Vertreter: LS-MP von Puttkamer Berngruber Loth Spuhler

(57) **Zusammenfassung**

Verfahren zur Planung und/oder zur Modifikation und/oder zur Steuerung und/oder zur Bereitstellung von Daten und/oder Informationen, die den Pflegeprozess eines Patienten betreffen. Die Daten und/oder Informationen werden in einem elektronischen Dokumentationssystem eines Eingabe-/Ausgabewerks einer Rechnereinheit abgespeichert. Die Daten und/oder Informationen werden zur weiteren Verarbeitung vom Eingabe-/Ausgabewerk wenigstens einer weiteren Komponente der Rechnereinheit zugeführt.

Es findet eine Interaktion mit der Pflegefachperson statt, indem sie in einer Phase der konzeptualisierten Wissensintegration (Synthese) Ergebnisse der automatisiert verarbeiteten Daten und/oder Informationen bewertet und nötigenfalls beeinflusst. Die veränderten Daten fließen zurück in die Rechnereinheit und werden dort für weitere Zwecke abgespeichert.

Die Planung und/oder die Modifikation und/oder die Steuerung und/oder die Bereitstellung der Daten und/oder Informationen, beruht auf Basis einer ereignisgesteuerten Navigation (Loop-Funktion). Die Daten und/oder Informationen unterliegen im Rahmen der ereignisgesteuerten Navigation der Analyse durch künstliche Intelligenz (KI). Die Daten und/oder Informationen beruhen auf einer Interaktion des elektronischen Dokumentationssystems mit wenigstens einer Pflegefachperson.

## Beschreibung

Die Erfindung betrifft ein Verfahren zur Planung und/oder zur Modifikation und/oder zur Steuerung und/oder zur Bereitstellung von Daten und/oder Informationen nach dem Oberbegriff des Anspruchs 1.

### Stand der Technik

Insbesondere in Krankenhäusern unterliegt die Dokumentation der Maßnahmen und/oder der Phasen des Pflegeprozesses einem kontinuierlichen Wandel. Die anfängliche Papierdokumentation geht über in eine computerisierte, formularbasierte Erfassung der Daten- und/oder Informationen.

Die zukünftige Erfassung der Daten- und/oder Informationen des Pflegeprozesses basiert auf der Nutzung agiler, digitaler Workflows.

Zur Steuerung der Abläufe des Pflegeprozesses im Pflegealltag sollten sich zukünftige moderne Lösungen mit Hilfe digitaler Workflows dynamisch an die jeweils aktuellen fachlichen Bedarfe der Pflegefachperson anpassen.

Die Gestaltung des Pflegeprozesses sowie die Dokumentation des Pflegeprozesses erfolgten bisher auf der Grundlage von sogenannten Regelkreismodellen oder sogenannten kybernetischen Modellen.

Auf die Regelkreismodelle und die kybernetischen Modelle im Rahmen der Pflege eines Menschen wird weiter unten im Detail eingegangen. Im Folgenden wird anstatt von einem Menschen allgemein vom Patienten gesprochen.

Die Aufteilung der Denk- und Handlungsabläufe des Pflegeprozesses in kleinere Schritte erleichtert beim bekannten Pflegeprozessmodell die Entwicklung eines standardisierten Vorgehens im pflegerischen Denken und Handeln.

Beim bekannten Pflegeprozessmodell wird die Komplexität des Alltags der Pflegefachperson nur ungenügend abgebildet. Das bislang bekannte Pflegeprozessmodell erscheint in der Praxis daher als unvollständig und realitätsfern.

Die Einteilung des Pflegeprozessmodells in Phasen ist für Pflegepraxis vielfach irrelevant. Bei derartigen Einteilungen handelt es sich vielfach um künstliche sowie nicht der Realität entsprechende Einteilung des Pflegeprozesses. Die bekannten Einteilungen der Pflegeprozesse entsprechen vielfach nicht der Realität, da die Pflegeprozesse dynamische Abläufe darstellen.

Bekannte Phasen des Pflegeprozesses, vorzugsweise, aber nicht ausschließlich, die Sammlung von Daten und/oder Informationen, die Diagnose, die Festlegung der Pflegemaßnahmen, die Durchführung der Pflegemaßnahmen und die Evaluation, können um weitere Phasen ergänzt werden. Die Ergänzung um weitere Phasen kann in Abhängigkeit von der konzeptionellen Festlegung der Pflegemaßnahmen erfolgen.

Im Vorfeld der Adaption der Pflegemaßnahmen sowie im Vorfeld der Ergänzung der Phasen des Pflegeprozesses hinterfragt die Pflegefachperson kontinuierlich die pflegerischen Erkenntnisse und Entscheidungen, die den Maßnahmen des Pflegeprozesses zugrunde liegen.

Die Phasen des Pflegeprozesses können überlappen. Die Phasen des Pflegeprozesses sind nicht in einer starren Abfolge zueinander angeordnet. Einzelne Phasen des Pflegeprozesses können entfallen oder auch wiederholt werden.

### Aufgaben der Erfindung

Die Erfindung stellt sich die Aufgabe, ein Verfahren für ein flexibles und anpassungsfähiges Modell des Pflegeprozesses bereitzustellen.

Es soll ein Verfahren zur Bereitstellung von Daten und/oder Informationen für den Pflegeprozess bereitgestellt werden, mit dem die Komplexität und Flexibilität der nichtlinearen Entscheidungsfindungsprozesse in eine elektronische Dokumentation integriert werden können.

Eine weitere Aufgabe besteht darin, ein Verfahren für ein Modell eines Pflegeprozesses vorzustellen, das eine flexible und kontextabhängige Dokumentation im Bereich der Pflege bereitstellt.

### Lösung

Die Aufgaben werden gelöst mit Hilfe eines Verfahrens zur Planung und/oder zur Modifikation und/oder zur Steuerung und/oder zur Bereitstellung von Daten und/oder Informationen, die den Pflegeprozess eines Patienten betreffen.

Die Daten und/oder Informationen sind in einem elektronischen Dokumentationssystem eines Eingabe-/Ausgabewerks einer Rechnereinheit abgespeichert.

Die Daten und/oder Informationen werden zur weiteren Verarbeitung vom Eingabe-/Ausgabewerk wenigstens einer weiteren Komponente der Rechnereinheit zugeführt.

Die Planung und/oder die Modifikation und/oder die Steuerung und/oder die Bereitstellung der Daten und/oder Informationen beruhen auf Basis einer ereignisgesteuerten Navigation.

Die Daten und/oder Informationen unterliegen im Rahmen der ereignisgesteuerten Navigation der Analyse durch künstliche Intelligenz (KI).

Die Daten und/oder Informationen beruhen auf einer Interaktion des elektronischen Dokumentationssystems mit wenigstens einer Pflegefachperson.

### Pflegeprozess

Nach aktuellem Stand der Wissenschaft beschreibt der Pflegeprozess in der beruflichen Gesundheits- und Krankenpflege ein systematisches Verfahren zur Erfassung des individuellen Pflegebedarfs, zur Planung, zur Durchführung und zur Evaluierung pflegerischer Maßnahmen an einem Patienten. Die Anzahl der einzelnen Phasen kann variieren, der Zweck bleibt immer gleich.

Die Umsetzung des Pflegeprozesses ist gemäß § 4 Abs. 2 PflBG eine Vorbehaltstätigkeit, die ausschließlich von Pflegefachpersonen ausgeführt werden darf.

Im § 4 Abs. 2 Nr. 1 - 3 PflBG regelt der Gesetzgeber die Bestandteile des pflegerischen Vorbehaltsrechts. Dabei handelt es sich um folgende Aufgaben:
- Erhebung und Feststellung des individuellen Pflegebedarfs
- Organisation, Gestaltung und Steuerung des Pflegeprozesses
- Analyse, Evaluation, Sicherung und Entwicklung der Qualität der Pflege

### Planung und/oder Modifikation und/oder Steuerung des Pflegeprozesses

Die "Planung" beschreibt die Fähigkeit und/oder Tätigkeit der Pflegefachperson zur gedanklichen Vorwegnahme der Phasen und/oder der Pflegemaßnahmen, die zur Erreichung der Ziele des Pflegeprozesses am Patienten notwendig sind. Die Planung der Pflegemaßnahmen erfolgt auf Basis einer zeitlich geordneten Menge von Daten und/oder Informationen über den Patienten.

Die "Modifikation" beschreibt die Veränderung und/oder die Umwandlung und/oder die Anpassung bestehender Sachverhalte und Gegebenheiten der Phasen des Pflegeprozesses.

Der Begriff "Steuerung" beschreibt die Einflussnahme auf die Phasen des Pflegeprozesses durch die Pflegefachperson. Die Veränderung des Pflegeprozesses erfolgt auf Grundlage von Daten und/oder Informationen, die vorzugsweise, aber nicht ausschließlich, von medizinischen Geräten abgerufen werden können.

### Regelkreismodell zur Steuerung und zur Regelung im bisherigen Pflegeprozessmodell

Das Regelkreismodell steuert und/oder regelt die Daten und/oder Informationen, die in dem elektronischen Dokumentationssystem des Eingabe-/Ausgabewerks der Recheneinheit abgespeichert sind.

Der Pflegeprozess beschreibt in der professionellen Gesundheits- und Krankenpflege ein systematisches Verfahren zur Erfassung und/oder zur Planung und/oder zur Durchführung und/oder zur Evaluierung pflegerischer Maßnahmen an einem Patienten.

Der Pflegeprozess nimmt dabei Bezug auf das Regelkreismodell des Pflegeprozesses, auf das weiter unten im Detail Bezug genommen wird.

Der aus dem Stand der Technik bekannte Pflegeprozess wird meist als ein linearer Ablauf beschrieben. Im Stand der Technik umfasst der Pflegeprozess vielfach eine oder mehrere der nachfolgend genannten Phasen: Problemfeststellung, Festlegung der erforderlichen Pflegemaßnahmen, Durchführung der Pflegemaßnahmen, Bewertung (Evaluation) des Erfolgs der Maßnahmen des Pflegeprozesses.

Das Regelkreismodell ist ein Konzept der Steuerung und der Regelung des Pflegeprozesses. Das Regelkreismodell des Pflegeprozesses kann aus mehreren Komponenten bestehen. Beispielhaft, aber in keiner Weise ausschließlich, umfasst das Regelkreismodell als Komponenten einen Regler und/oder ein Stellglied und/oder eine Regelstrecke und/oder einen Sensor. Das Regelkreismodell dient dazu, einen Sollwert mit einem Istwert zu vergleichen. Der Soll-Ist-Vergleich des Regelkreismodells soll Abweichungen innerhalb des Pflegeprozesses korrigieren, um stabile Systemzustände effizienter gestalten und präziser steuern zu können.

Das Regelkreismodell mit seinem Regelkreis wird auch als Synonym für den Pflegeprozess als solchen verstanden. Dabei leitet das Regelkreismodell die Pflegefachperson an, den Pflegeprozess strukturiert zu steuern. Die Steuerung des Pflegeprozesses erfolgt vorzugsweise, aber nicht ausschließlich, durch Soll- bzw. Zielvorgaben. Darüber hinaus kann die Steuerung durch einen abschließenden Ist-Soll-Vergleich strukturiert gesteuert werden.

Das bisherige Regelkreismodell basiert auf der Annahme, dass der Pflegeprozess ein Problemlösungsprozess und/oder ein Beziehungsprozess ist, der sich zwischen der Pflegefachperson und dem Patienten vollzieht.

Der Pflegeprozess wird über wenigstens einen Rückkopplungsprozess gesteuert. Vorzugsweise, aber nicht ausschließlich, umfasst der Pflegeprozess vier bis sechs Phasen. Die Phasen bauen logisch aufeinander auf und können sich gegenseitig beeinflussen.

Die Phasen des Pflegeprozesses werden in der gesetzlich vorgeschriebenen Pflegedokumentation abgebildet. Auf die Pflegedokumentation wird weiter unten im Detail eingegangen.

Rein beispielhaft wird im Folgenden das vierphasige Modell sowie das sechsphasige Modell näher beschrieben.

### Regelkreismodell - vierphasiges Modell

Im vierphasigen Modell wird der Pflegebedarf des Patienten eingeschätzt und dazu eine Sammlung aller pflegerelevanten Informationen erstellt.

Im Rahmen der Erstellung des Pflegeplans wird eine Pflegediagnose erstellt. Pflegeprobleme und Ressourcen zur Durchführung der Pflegemaßnahmen werden einander gegenübergestellt. Pflegeziele werden bestimmt und Pflegemaßnahmen darauf abgestimmt und geplant.

Im Rahmen der praktischen Ausführung des Pflegeplans können zusätzliche Interventionen und/oder weitere Implementationen erforderlich werden.

Die Wirkung und die Qualität des Pflegeprozesses werden beurteilt. Hierzu wird überprüft, ob die Maßnahmen des Pflegeprozesses die gesetzten Ziele erreicht haben und ob die Pflegequalität ausreichend war.

### Regelkreismodell - sechsphasiges Modell

Im sechsphasigen Modell wird der Pflegebedarf des Patienten eingeschätzt (Pflegeanamnese). Dazu wird eine Sammlung aller pflegerelevanten Informationen erstellt.

Im Rahmen der Erstellung des Pflegeplans wird eine Pflegediagnose erstellt. Pflegeprobleme und Ressourcen zur Durchführung der Pflegemaßnahmen werden einander gegenübergestellt.

In einem separaten Schritt werden die Pflegeziele bestimmt.

Die Pflegemaßnahmen werden auf die Pflegeziele abgestimmt und geplant.

Die geplanten Pflegemaßnahmen werden durchgeführt.

Die Wirkung der durchgeführten Maßnahmen wird beurteilt. Es wird geprüft, ob die vorab festgelegten Ziele und damit die geplante Pflegequalität erreicht wurden.

### Kybernetisches Modell

Das sogenannte kybernetische Modell trägt dazu bei, mithilfe mathematischer oder visueller symbolischer Darstellungen der Komponenten (z. B. Regler; Stellglied; Sensor) die Auswirkungen von Rückmeldungen auf die potenziellen Organisations- oder Leistungszustände zu verstehen.

### Pflegeprozess als Outcome-Present State-Test Model for Reflective Practice

Das sogenannte Outcome-Present State-Test Model for Reflective Practice (im Folgenden: OPT) ist ein weiteres Modell eines Pflegeprozesses. Das OPT basiert auf einer gleichartigen und nichtlinearen Informationsverarbeitung. Die Informationsverarbeitung kann mehrfach wiederholt werden (iterativ und rekursiv).

Die Verarbeitung der Daten und/oder Informationen, die auf den Pflegeprozess bezogen sind, kann iterativ erfolgen. Die iterative Verarbeitung der auf den Pflegeprozess bezogenen Daten und/oder Informationen bedeutet, dass die Verarbeitung der Daten und/oder Informationen wiederholt, in gleicher oder ähnlicher Weise erfolgt. Die iterative Verarbeitung der auf den Pflegeprozess bezogenen Daten und/oder Informationen, hat eine Optimierung der Phasen und/oder Maßnahmen des Pflegeprozesses zum Ziel.

Das OPT macht der Pflegefachperson die für den Patienten vorgesehenen Pflegemaßnahmen in ihrer gesamten Komplexität zugänglich.

Das OPT umfasst die Unterstützung der klinischen Begründung der jeweiligen Pflegemaßnahme des Pflegeprozesses durch die Reflexion und den gezielten Einsatz kognitiver sowie metakognitiver Fähigkeiten der jeweiligen Pflegefachperson.

Der Begriff "klinische Begründung" (Clinical Reasoning) ist aus dem Bereich der Medizin sowie aus dem Bereich der Therapiewissenschaften bekannt. Die sogenannte klinische Begründung kann auch als "klinische Schlussfolgerung" bezeichnet werden. Der Begriff "klinische Begründung" bezeichnet in Bezug auf den Pflegeprozess alle Denk- und/oder Handlungs- und/oder Entscheidungsprozesse, die in der täglichen Arbeit von Ärzten und/oder Therapeuten und/oder Pflegepersonal anfallen.

Das OPT verknüpft die gesundheitlichen Probleme des Patienten miteinander. Miteinander konkurrierende Diagnosen des Patienten sowie Wechselwirkungen zwischen den ausgewählten Pflegemaßnahmen werden einer gesamtheitlichen Betrachtung unterzogen.

Mit der gesamtheitlichen Betrachtung erkennt die Pflegefachperson den Zusammenhang zwischen der Dynamik der Patientenbedürfnisse auf einerseits und den kausalen und/oder korrelativen Zusammenhängen zwischen den einzelnen Maßnahmen des Pflegeprozesses andererseits.

### Pflegefachperson

Unter dem Begriff "Pflegefachperson" sind sämtliche Pflegefachfrauen und Pflegefachmänner gemäß § 1 PflBG zu verstehen.

Vorzugsweise, aber nicht ausschließlich, arbeitet die Pflegefachperson in der Altenpflege und/oder in der Gesundheitspflege und/oder in der Krankenpflege. Es versteht sich von selbst, dass die Pflegefachperson auch in der Kinderkrankenpflege und/oder in der Intensiv-Fachpflege arbeiten kann.

Die Pflegefachperson kann in einem Pflegeheim und/oder im ambulanten Pflegedienst arbeiten.

Das Wissen der Pflegefachperson basiert auf Kenntnissen, die während der pflegerischen Berufsausbildung vermittelt wurden. Die Kenntnisse der Pflegefachperson umfasst zusätzlich ihre praktischen Berufserfahrungen, die sich die Pflegefachperson im Rahmen ihrer Berufstätigkeit aneignen konnte.

Das Wissen der Pflegefachperson umfasst zusätzlich sogenanntes implizites pflegerisches Wissen. Zu dem impliziten Wissen der Pflegefachperson gehören Sachverhalte, die für die Pflegefachperson nicht aus sich selbst heraus zu verstehen sind. Vielmehr muss sich die Pflegefachperson das implizite Wissen während der praktischen Tätigkeit, während des Pflegeprozesses am Patienten logisch selbst erschließen. Komplexe und/oder dynamische und/oder unvorhersehbare Situationen des Pflegealltags, fordern der Pflegefachperson im Rahmen der Aneignung impliziten Wissens eine flexible und/oder anpassungsfähige Herangehensweise ab.

Die erfahrene Pflegefachperson verfügt über eine ausgeprägte Fachkompetenz. Die Pflegefachperson erkennt auf Grund ihrer Expertise die gesundheitlichen Probleme des Patienten intuitiv, bevor manifeste Symptome auftreten.

Die intuitive Wahrnehmung der Pflegefachperson basiert auf Erfahrung und/oder klinischem Wissen in Verbindung mit der kontinuierlichen Beobachtung der Patienten.

Das neue Verfahren zur Planung und/oder zur Modifikation und/oder zur Steuerung des Pflegeprozesses eines Patienten sowie zur Bereitstellung von Daten und/oder Informationen, integriert die intuitive Wahrnehmung der Pflegefachperson in die digitale Dokumentation der Daten und/oder Informationen des Pflegeprozesses.

Das neue Verfahren kombiniert die alltägliche pflegerische Erfahrung der Pflegefachperson mit ihrem klinischen Wissen. Die pflegerische Erfahrung und das klinische Wissen werden darüber hinaus kombiniert mit den fortwährenden Beobachtungen des Patienten durch die Pflegefachperson. Die Kombination der alltäglichen pflegerischen Erfahrung mit klinischem Wissen sowie den Beobachtungen des Patienten durch die Pflegefachperson bezeichnet das neue Verfahren als kontextualisierte Wissensintegration.

Das derzeitige Verfahren fordert die Pflegefachperson auf, ihr Handeln zu begründen, bevor sie in die Maßnahmenplanung oder -durchführung geht. Voraussetzung für eine derartige Initiierung durch die Pflegefachperson sind vorherige eindeutige Symptome des Patienten oder standardisierte Dokumentationsfelder, die die Pflegefachperson zu einer entsprechenden Maßnahme auffordern. Die Initiierung einer Phase des Pflegeprozesses und/oder die Initiierung einer pflegerischen Maßnahme durch die Pflegefachperson, erfordert von der Pflegefachperson eine sogenannte Pflegediagnose. Die Pflegediagnose kann softwaregestützt und/oder automatisiert sein.

Das Sammeln des intuitiven Wissens der Pflegefachperson im Rahmen des neuen Verfahrens zur Planung und/oder zur Modifikation und/oder zur Steuerung eines Pflegeprozesses eines Patienten sowie zur Bereitstellung von Daten und/oder Informationen, wird mit Hilfe einer ereignisgesteuerten Navigation umgesetzt.

Das Sammeln des intuitiven Wissens der Pflegefachperson im Rahmen des neuen Verfahrens eröffnet die Möglichkeit, das Wissen der jeweiligen Pflegefachperson in die künftige Entwicklung und/oder in das künftige Training künstlicher Intelligenz-Modelle (KI-Modelle) zu integrieren.

Die Pflegefachperson verfügt über wertvolle, oftmals unbewusste pflegebezogene Erkenntnisse und Erfahrungen. Die pflegebezogenen Erkenntnisse und Erfahrungen spielen in der täglichen Praxis der Pflegefachperson eine entscheidende Rolle. Ein gravierender Nachteil des bisherigen Vorgehens liegt jedoch darin, dass die pflegebezogenen Erkenntnisse und Erfahrungen der Pflegefachperson häufig nicht explizit dokumentiert sind.

Die pflegebezogenen Erkenntnisse und Erfahrungen der Pflegefachperson können somit nicht großflächig in Umlauf gebracht werden. Eine systematische Erfassung und/oder Analyse des intuitiven Wissens der Pflegefachperson wurde den interessierten Fachkreisen für die Praxis und/oder für die Schulung künftiger Pflegekräfte bislang nicht zugänglich und nutzbar gemacht.

Im neuen Verfahren können auf Grundlage der pflegebezogenen Erkenntnisse und Erfahrungen der Pflegefachperson KI-Modelle trainiert werden. Derartig trainierte KI-Modelle sind in der Lage, gesundheitliche Komplikationen bei Patienten frühzeitig und/oder präzise zu erkennen.

Auf diese Weise wird die Qualität der pflegerischen Versorgung erhöht. Eine effizientere und/oder proaktive Versorgung des Patienten kann somit sichergestellt werden.

Potenziellen Risiken und/oder möglichen Komplikationen kann bereits im Frühstadium begegnet werden.

### Medizinische Geräte

Medizinische Geräte bilden einen erheblichen Teil aller Medizinprodukte. Auch die Dokumentationssoftware, in der das neue Verfahren technisch umgesetzt und in Verkehr gebracht wird, ist als ein solches Medizinprodukt einzuordnen.

Vorzugsweise, aber nicht ausschließlich, werden medizinische Geräte von der Pflegefachperson im Rahmen des Pflegeprozesses eingesetzt. Die Pflegefachperson bringt medizinische Geräte am Patienten zur Anwendung. Während des Pflegeprozesses überwacht und/oder kontrolliert die Pflegefachperson die Funktionsweise des medizinischen Gerätes am Patienten.

Die Pflegefachperson bestimmt darüber hinaus die Dauer des Einsatzes der medizinischen Geräte am Patienten. Es versteht sich von selbst, dass die Pflegefachperson im Hinblick auf medizinische Geräte auch andere Aufgaben ausführen kann.

Die Pflegefachperson überwacht die Daten und/oder die Informationen, die von medizinischen Geräten erzeugt werden. Die Auswertung und/oder die weitere Be- und Verarbeitung der Daten und/oder der Informationen, kann ebenfalls in den Aufgabenbereich der Pflegefachperson fallen.

Vorzugsweise, aber nicht ausschließlich, werden medizinische Geräte in Praxen und in Kliniken eingesetzt. Medizinische Geräte können aber auch zu Lehrzwecken in Ausbildungsstätten und/ oder Universitäten eingesetzt werden.

Die medizinischen Geräte dienen der Diagnostik und/oder der Vorbeugung einer Krankheit und/oder auch der Therapie der jeweiligen Krankheit.

Vorzugsweise, aber nicht ausschließlich, werden medizinische Geräte in allen Bereichen eingesetzt, in denen pflegerische Versorgung stattfindet, wie z. B., aber nicht ausschließlich, im Krankenhaus, Psychiatrie, Pflegeheim, Rehabilitationseinrichtung oder ambulante Pflege.

### Rechnereinheit mit Dokumentationssystem zur elektronischen Dokumentation des Workflows des Pflegeprozesses

Die Rechnereinheit bezeichnet einen Rechner, vorzugsweise, aber nicht ausschließlich, einen Computer. Die Rechnereinheit ist ein Gerät, das mittels programmierbarer Rechenvorschriften Daten verarbeitet.

Vorzugsweise, aber nicht ausschließlich, umfasst die Rechnereinheit (Computer) ein Rechenwerk. Die Rechnereinheit weist darüber hinaus eine oder mehrere der folgenden Komponenten auf: ein Steuerwerk und/oder eine Buseinheit und/oder ein Speicherwerk und/oder ein Eingabe-/Ausgabewerk.

Die Daten und/oder Informationen werden wenigstens einer Komponente der Rechnereinheit vom Eingabe-/Ausgabewerk zur weiteren Verarbeitung zugeführt.

Das Steuerwerk der Rechnereinheit erhält Daten und/oder Informationen aus einem Arbeitsspeicher der Rechnereinheit.

Die Befehle werden im Steuerwerk interpretiert. Die Daten und/oder Informationen werden im Rechenwerk verarbeitet.

Das Speicherwerk der Rechnereinheit umfasst einen sogenannten RAM-Speicher (RAM) und eine Speicherlogik, die in einem Prozessor abgelegt ist.

Das Eingabe-/Ausgabewerk der Rechnereinheit umfasst die Datenspeicher. Als Datenspeicher dient beispielhaft, aber nicht ausschließlich, wenigstens eine Festplatte. Zur weiteren Verarbeitung werden Daten und/oder Informationen von der Festplatte in einen Arbeitsspeicher geladen.

Datenspeicher, vorzugsweise, aber nicht ausschließlich, die Festplatten, gehören nicht zum Speicherwerk, sondern zum Eingabe-/Ausgabewerk. Die Daten werden von der Festplatte zunächst in den Arbeitsspeicher geladen. Vor der nachfolgenden Verarbeitung werden die Daten und/oder Informationen vom Arbeitsspeicher wenigstens einer weiteren Komponente der Rechnereinheit zugeführt.

Vorzugsweise, aber nicht ausschließlich, umfasst das Eingabe-/Ausgabewerk der Rechnereinheit (Computer) ein elektronisches Dokumentationssystem.

Das Eingabe-/Ausgabewerk der Rechnereinheit kommuniziert zum Empfang und/oder zur Übermittlung von Daten und/oder Informationen mit wenigstens einem medizinischen Gerät.

Das sogenannte Krankenhausinformationssystem (KIS) umfasst die Gesamtheit aller informationsverarbeitenden Systeme der Informationstechnik zur Erfassung, Bearbeitung und Weitergabe medizinischer und administrativer Daten und/oder Informationen im Krankenhaus.

Das elektronische Dokumentationssystem ist zum Abspeichern von Daten und/oder Informationen vorgesehen, die im Zusammenhang mit der Pflege von Patienten stehen.

Das elektronische Dokumentationssystem integriert die Daten und/oder Informationen des bisherigen Pflegeprozessmodells in das elektronische Dokumentationssystem des Verfahrens zur Planung und/oder zur Modifikation und/oder zur Steuerung eines Pflegeprozesses eines Patienten sowie zur Bereitstellung von Daten und/oder Informationen.

Das Dokumentationssystem basiert bisher auf einem sequenziellen Dokumentationsworkflow.

Der aus dem Stand der Technik bekannte sequenzielle Dokumentationsworkflow erlaubt keine dynamische Anpassung an sich ändernde Pflegebedürfnisse. Hierdurch entsteht der jeweiligen Pflegefachperson ein zusätzlicher Arbeitsaufwand.

Vorzugsweise, aber nicht ausschließlich, werden bekannte Pflegeprozessmodelle als lineare Abläufe dargestellt. Die bekannten Pflegeprozessmodelle umfassen vorzugsweise, aber nicht ausschließlich, die Phasen "Feststellung des Pflegebedarfs" und/oder "Planung" und/oder "Durchführung" und "Evaluation der Pflegemaßnahmen" (nachfolgend beispielhaft mit A-B-C-D dargestellt).

Diese Methodik mit ihrer vorgegebenen Reihenfolge ist in vielen elektronischen Pflegedokumentationssystemen weit verbreitet. Sie basiert auf der Annahme, dass Pflegeinterventionen und Pflegeentscheidungen in einer vorhersagbaren, sequenziellen Weise stattfinden. Dadurch ist die Pflegefachperson in vorgegebenen, starren Dokumentationsabläufen gefangen, was aber nicht den tatsächlichen kognitiven Vorgängen einer Pflegefachperson entspricht. In ihrer klinischen Entscheidungsfindung im Rahmen des Pflegeprozesses geht sie nicht stur von A-B-C-D, sondern springt zum Beispiel von A nach D weiter nach C und zurück nach A. Diese Diskrepanz zwischen Nutzeranforderung und tatsächlicher Umsetzung im bisherigen Pflegedokumentationssystem kann zu einem negativen Nutzererlebnis (abgekürzt: UX; user experience) führen.

Weiterhin umfasst das elektronische Dokumentationssystem Daten und/oder Informationen, die die Benutzerschnittstelle in Form einer Benutzeroberfläche (abgekürzt: UI; user interface) des elektronischen Dokumentationssystems betreffen. Im Rahmen der pflegerischen Arbeit tritt die Pflegefachperson über die Benutzeroberfläche mit dem elektronischen Dokumentationssystem in Kontakt.

Im neuen Verfahren ist eine intelligente, ereignisgesteuerte Benutzeroberfläche vorgesehen, über die die Interaktion zwischen dem elektronischen Dokumentationssystem und der Pflegefachperson erfolgt.

Rein beispielhaft, aber nicht ausschließlich, bezeichnet die UI des neuen Verfahrens die intelligente Benutzeroberfläche eines Startbildschirms. Zusätzlich können Knöpfe an einem Smartphone eine Benutzerschnittstelle darstellen. Mit der Benutzerschnittstelle kann eine bestimmte Funktion angesteuert werden. Die UI kann außer einer reinen Softwarekomponente auch eine Hardwarekomponente sein.

Das elektronische Dokumentationssystem dient der Integration der Daten und/oder Informationen in Krankenhausinformationssysteme (KIS) für das neue Verfahren eines nichtlinearen Pflegeprozesses. Das neue Verfahren basiert dabei auf flexiblen und/oder iterativen Entscheidungs- und Handlungsprozessen. Mit dem neuen Verfahren ermöglicht das elektronische Dokumentationssystem der Pflegefachperson, die Phasen des Pflegeprozesses nichtlinear anzusteuern.

Dafür kann das elektronische Dokumentationssystem wenigstens zwei sogenannte Feedback-Schleifen umfassen. Mit Hilfe der Feedback-Schleifen kann das elektronische Dokumentationssystem die Phasen des Pflegeprozesses in Echtzeit anpassen.

Das elektronische Dokumentationssystem stellt der Pflegefachperson eine praxisorientierte und/oder technisch realisierbare Lösung zur Seite, um das implizite Wissen der Pflegefachperson vorzugsweise, aber nicht ausschließlich, in vollem Umfang in Krankenhausinformationssystemen berücksichtigen zu können.

Bisher bekannte elektronische Dokumentationssysteme für Daten und/oder Informationen im Pflegeprozess basieren vielfach auf einer strukturierten, aber starren Vorgehensweise. Hierdurch wird ein dynamischer, nichtlinearer Verlauf eines modernen Pflegeprozesses nur unzureichend abgebildet.

Bekannten elektronischen Dokumentationssystemen für Daten und/oder Informationen des Pflegeprozesses steht bei der Dokumentation der Daten und/oder Informationen vielfach nur eine eingeschränkte Flexibilität zur Verfügung. Diejenigen Daten und/oder Informationen, die von der Pflegefachperson beigesteuert werden könnten, bleiben oftmals außer Betracht.

Bisher bekannte elektronische Dokumentationssysteme für Daten und/oder Informationen können im Rahmen der Pflege des Patienten bei nichtlinearen Pflegeprozessen keine oder nur unzureichende Unterstützung zur Verfügung stellen.

Bei der Arbeit mit bekannten elektronischen Dokumentationssystemen für Daten und/oder Informationen muss die Pflegefachperson zwischen verschiedenen Dokumenten und/oder Eingabemasken hin- und herwechseln.

Die Pflegefachperson verliert dadurch wertvolle Arbeitszeit und es entsteht ein erhöhter Arbeitsaufwand, der der Pflege des Patienten nicht zugutekommt.

Die Eingabe und die Verarbeitung alternativer und/oder paralleler und/oder wiederholt auftretender Daten und/oder Informationen werden bei bekannten elektronischen Dokumentationssystemen für Daten und/oder Informationen nicht optimal gelöst.

Das elektronische Dokumentationssystem kommuniziert mit externen Pflegesystemen.

### Neues Verfahren zur Planung und/oder zur Modifikation und/oder zur Steuerung und/oder zur Bereitstellung von Daten und/oder Informationen, die einen Pflegeprozess eines Patienten betreffen

### Verfahren - Workflow des Pflegeprozesses

Das vorgeschlagene neue Verfahren zur Planung und/oder zur Modifikation und/oder zur Steuerung des Workflows des Pflegeprozesses eines Patienten umfasst die folgenden Phasen. Die nachfolgende Nummerierung der Phasen wurde nur zur besseren Übersichtlichkeit und Nachvollziehbarkeit gewählt, stellt aber keine sequenzielle Reihenfolge der einzelnen Prozessschritte dar.

**REFLEXIVE EINSCHÄTZUNG:** Eine erste Phase leitet den Pflegeprozess ein. Sie ist geprägt von einer umfassenden, strukturierten und standardisierten Einschätzung des (pflegerelevanten) Gesundheitsstatus des Patienten. Die Einschätzung des Gesundheitsstatus basiert sowohl auf einem expliziten als auch auf einem impliziten Wissen bzw. Kenntnisstand der Pflegefachperson.

In der ersten Phase des Pflegeprozesses kommen theoretische Instrumente der Pflegefachperson wie zum Beispiel, aber nicht ausschließlich strukturierte und standardisierte Assessmentinstrumente zum Einsatz (z. B. die Instrumente epaAC für die stationäre Akutversorgung [Acute Care], epaKIDS für die pädiatrische Akutversorgung, epaMHC für die psychiatrische Versorgung [Mental Health Care], epaLTC für die stationäre Langzeitversorgung [LongTermCare])

Die Einschätzung des Gesundheitsstatus des Patienten erfolgt als kontinuierlicher Reflexionsprozess, der regelmäßig während des Pflegeprozesses durchgeführt wird. Zur Einschätzung gehört auch die Identifikation pflegetherapeutisch relevanter Beeinträchtigungen des Gesundheitszustands des Patienten, für die die Pflegefachperson ein explizites zu erreichendes Zielniveau festlegt.

**KONTEXTUALISIERTE WISSENSINTEGRATION (SYNTHESEBILDUNG):** In der zweiten Phase erfolgt eine Kontextualisierung aller bisherigen gewonnenen Informationen, ergänzt um Daten aus medizintechnischen Geräten, wie z. B., aber nicht ausschließlich Bewegungssensoren, Temperatursonden oder Smart Clothes. Außerdem erhält die Pflegefachperson die Möglichkeit, neben dem regelbasierten expliziten (theoretischen) Wissen ihr implizites Erfahrungswissen einzubringen und zu bewerten (Synthesebildung). Diese explizite Synthesebildung als visualisierter und interaktiver Schritt stellt ein neues Element im Pflegeprozess dar.

Anstelle eines festen Ablaufs der Phasen des Pflegeprozesses kann in Abhängigkeit von der Kontextualisierung (z. B. wenig komplexer Patientenfall) übersprungen und direkt in die Maßnahmenplanung gesprungen werden (Zeitersparnis!).

Zur kontextualisierten Wissensintegration werden die Daten und/oder die Informationen aus der Einschätzung des Gesundheitszustands des Patienten sowie aus dazugehörigen automatisierten Regelableitungen der Pflegediagnosen abgeleitet. Die Pflegediagnosen umfassen eine oder mehrere automatisch, nach festgelegtem Regelwerk gebildete Risikodiagnose(n) und/oder eine oder mehrere Basisdiagnose(n) und/oder eine oder mehrere Fokusdiagnose(n). Fokusdiagnosen werden automatisch gebildet, sobald die Pflegefachperson mindestens ein Zielniveau festlegt.

Zur kontextualisierten Wissensintegration werden die Daten und/oder die Informationen automatisiert aufbereitet, um aus einer sogenannten Ressourcenableitung weitere relevante Kontextinformationen für die Entscheidungsfindung, welche Pflegemaßnahmen für den individuellen Patienten erforderlich sind, zur Verfügung zu stellen.

In der zweiten Phase der kontextualisierten Wissensintegration (Synthesebildung) wird unbewusstes und/oder intuitives (implizites) Wissen der Pflegefachperson in eine klare, verständliche und nachvollziehbar dokumentierte Form überführt. Das implizite Wissen der Pflegefachperson basiert dabei auf persönlichen Erfahrungen und/oder Fähigkeiten und/oder Einsichten der Pflegefachperson. Auf diese Weise findet das implizite Wissen der Pflegefachperson Eingang in die klinische Begründung der gewählten Pflegemaßnahmen.

Zur weiteren Kontextualisierung werden neben der Darstellung der festgelegten Zielwertniveaus zusätzliche Daten von Medizingeräten (wie z. B., aber nicht ausschließlich, Bewegungssensoren, Temperatursonden oder Smart Clothes.) sowie die Ergebnisse weiterer Messungen des pflegerelevanten Gesundheitsstatus integriert und am Bildschirm visualisiert, um alle relevanten Informationen anzuzeigen, die die Pflegefachperson bei der Entscheidungsfindung für die individuell notwendigen Pflegemaßnahmen benötigt.

Das Verfahren überprüft automatisch, ob die unterschiedlichen Datenquellen (Informationen aus Phase eins, externe Pflegesysteme [Fokusassessments] und/ oder Daten aus Medizingeräten zum gleichen Ergebnis [z. B. "Dekubitusrisiko erhöht"]) kommen oder nicht. Je nach Datenkonstellation wird die Pflegefachperson auf das Ergebnis hingewiesen, wobei es drei Ergebnisdimensionen gibt: Keine Datenquelle gibt ein Risiko aus, alle Datenquellen geben ein Risiko aus, die Datenquellen kommen zu unterschiedlichen Ergebnissen. Durch die intelligente Nutzeroberfläche werden entsprechende Symbole angezeigt. Bei differenten Ergebnissen wird die Pflegefachperson aufgefordert, eine Entscheidung auf Basis ihrer Fachexpertise zu treffen.

In dieser Phase kann die Pflegefachperson auch - gestützt auf das implizite Wissen resp. unter Berücksichtigung der Kontextinformationen - die Ergebnisse von standardisierten und/ oder automatisierten Risikoeinschätzungen überschreiben.

Sofern die Pflegefachperson in der zweiten Phase (kontextualisierte Wissensintegration) erkennt, dass sie weitere Ziele festlegen möchte oder bereits in der ersten Phase festgelegte Ziele anpassen oder streichen möchte, kann sie dies direkt in der zweiten Phase tun, ohne in die erste Phase zurückspringen zu müssen.

**ADAPTIVE PLANUNG:** In der dritten Phase des Verfahrens erfolgt eine adaptive Planung der pflegerischen Maßnahmen für den Patienten als Teil des Pflegeprozesses. Bei der adaptiven Planung werden die pflegerischen Maßnahmen flexibel gestaltet und an den jeweiligen Bedarf des Patienten angepasst.

Bei der adaptiven Planung der Maßnahmen des Pflegeprozesses werden objektive Daten und/oder Informationen, wie insbesondere Pflegediagnosen, Zielwertniveaus und Therapiepläne berücksichtigt. Zusätzlich fließen die Erfahrungen und/oder Einschätzungen der Pflegefachperson in die Planung der Maßnahmen des Patienten ein, die nicht standardisiert sind.

**MABNAHMENDURCHFÜHRUNG:** Die vierte Phase des Verfahrens sieht die Durchführung bzw. Verrichtung einer pflegerischen Maßnahme durch die Pflegefachperson vor. Die Maßnahme kann dabei vorab geplant sein oder durch ein Ad-hoc-Ereignis ausgelöst werden. Die durchgeführten Maßnahmen werden durch die Pflegefachperson dokumentiert bzw. die Verrichtung dieser quittiert.

**Ereignisgesteuerte Navigation LOOP-Funktion:** Die fünfte Phase des Verfahrens sieht eine ereignisgesteuerte Navigation mittels einer Loop-Funktion vor.

Die Loop-Funktion nutzt eine ex ante erstellte Verknüpfung zwischen den Beschreibungen des pflegerelevanten Gesundheitszustands und jenen Pflegemaßnahmen, die zu dem jeweiligen Gesundheitszustand passen. Beispielhaft sei hier der Zustand "stark beeinträchtigte Fähigkeit zur Veränderung der Körperposition im Bett" genannt, der mit verschiedenen Pflegemaßnahmen, wie beispielhaft, aber nicht ausschließlich, den Lagerungsmaßnahmen "Seitenlagerung", "Bauchlagerung", "Rückenlagerung" verknüpft ist. Diese Verknüpfungen liegen in Form von Datenmodellen vor, die im elektronischen Dokumentationssystem gespeichert sind.

Die Loop-Funktion führt dazu, dass die Schritte bei der Dokumentation des Pflegeprozesses nicht durch theoretische, sequenzielle Abläufe vorgegeben werden, sondern durch relevante Ereignisse gesteuert werden. Zu den relevanten Ereignissen zählen z. B. Änderungen des Gesundheitszustands des Patienten und/oder unvorhergesehene Komplikationen oder Anpassungen der pflegerischen Maßnahmen an sich. Das bedeutet, dass die angebotene Benutzeroberfläche für die Prozessdokumentation mittels Soll-Ist-Vergleich genauso vom Gesundheitszustand wie von der Maßnahme her gesteuert werden kann ("von vorne nach hinten" oder "von hinten nach vorne").

Beispielhaft, aber nicht ausschließlich, können Phasen des Pflegeprozesses also zurückgeführt werden. Das Verfahren stellt eine Darstellung bereit, die diejenigen Phasen und/oder Maßnahmen des Pflegeprozesses anzeigt, zwischen denen jeweils eine Verknüpfung im Datenmodell besteht, weil sie fachlich möglich und sinnvoll wäre, die jedoch im Pflegeprozess des Patienten nicht eingeleitet bzw. nicht durchgeführt wurden.

Die fünfte Phase des Verfahrens wird anhand eines Beispiels näher beschrieben.

Initial ist der Patient nicht beeinträchtigt in seiner Fähigkeit, die Körperposition zu verändern. Damit ist auch das Risiko für einen Dekubitus (Wundliegen) niedrig. Nun verändert sich die Situation: der Patient entwickelt starke Schmerzen und bewegt sich im Bett nicht mehr. Die Pflegefachperson erkennt dies und leitet Lagerungsmaßnahmen ein, um das Dekubitusrisiko zu reduzieren. Die Pflegefachperson dokumentiert im Anschluss die durchgeführte Maßnahme "Seitenlagerung durchführen". Das Verfahren prüft, ob der mit der Maßnahme verknüpfte Gesundheitszustand ("stark beeinträchtigte Fähigkeit zur Veränderung der Körperposition im Bett") bereits dokumentiert ist oder nicht (Loop-Funktion). Das bedeutet, dass zunächst geprüft wird, ob zu der durchgeführten Maßnahme bereits ein passender Eintrag zum pflegerelevanten Gesundheitszustand aus der ersten Phase (Reflexive Einschätzung) vorhanden ist. Falls ja, erfolgt keine weitere Aktion. Falls nein, wird die Pflegefachperson darauf hingewiesen, dass zu der Maßnahme noch keine passende Diagnose/ Problembeschreibung existiert. Nun entscheidet sie, ob sie diesen Schritt gehen und die zugehörige Diagnose/ Problembeschreibung dokumentieren will oder nicht. Wenn sie sich im o.g. Beispiel entscheidet, in die erste Phase zu springen, dokumentiert sie dort den veränderten Gesundheitszustand. Das Verfahren errechnet aus den neuen Daten automatisch, dass jetzt ein Dekubitusrisiko vorliegt. Dadurch wird das implizite Wissen der Pflegefachperson, die direkt gehandelt hat, ohne den sequenziellen diagnostischen Prozess zuvor schriftlich zu dokumentieren, eingebunden. Die veränderte Situation wird transparent und alle am Behandlungsprozess Beteiligten sind informiert.

Dieser dynamische und bidirektionale Ansatz zwischen den Phasen ist einzigartig. Frühere Prozessmodelle sahen ausschließlich den Weg vom Zustand zur Planung zur Maßnahme vor. Die im Fallbeispiel geschilderte Situation hätte mit früheren sequenziellen Prozessmodellen dazu geführt, dass das neu entstandene Dekubitusrisiko nicht dokumentiert worden wäre und ggf. für Dritte unerkannt geblieben wäre (Stichwort: Patientensicherheit).

Der Einsatz der Loop-Funktion im Prozess wird für alle Anwender automatisch durch ein optisches Symbol kenntlich gemacht.

Dieser dynamische bidirektionale Ansatz ermöglicht es, dass Daten und/oder Informationen im Pflegeprozess transparent nachvollzogen werden - eine wichtige Voraussetzung für den nächsten Schritt: Einbindung von KI zur weiteren Verbesserung der Patientensicherheit.

### Verbesserung der Patientensicherheit durch Einbindung von KI (Künstlicher Intelligenz) in die Loop-Funktion

Der digitale Workflow stellt für die klinische Entscheidungsfindung ein Frühwarnsystem bereit. Der digitale Workflow erkennt potenzielle Komplikationen frühzeitig. Proaktive Maßnahmen innerhalb des Pflegeprozesses können frühzeitig eingeleitet werden.

Daten und/oder Informationen, die auf Grundlage des digitalen Workflows entstehen, können Trainingszwecken und/oder der kontinuierlichen Verbesserung künftiger KI-gestützter Modelle und/oder der Optimierung klinischer Entscheidungsfindung dienen.

Hierzu analysiert die künstliche Intelligenz (KI) im Rahmen der ereignisgesteuerten Navigation die Daten und/oder Informationen auf Abweichungen gegenüber vorbestimmten Regeln und Algorithmen.

Das vorgeschlagene Verfahren zur Planung und/oder zur Modifikation und/oder zur Steuerung des Workflows des Pflegeprozesses eines Patienten, trägt zu einer sichereren und/oder einer effizienteren und/oder einer patientenorientierteren Pflege des Patienten bei, denn die Daten und/oder die Informationen können im Rahmen der ereignisgesteuerten Navigation der Analyse durch künstliche Intelligenz (KI) unterliegen.

Im Rahmen des Verfahrens zur Planung und/oder zur Modifikation und/oder zur Steuerung des Pflegeprozesses eines Patienten sowie zur Bereitstellung von Daten und/oder Informationen wird die ereignisgesteuerte Navigation in den folgenden Schritten technisch umgesetzt:
In einem ersten Schritt werden Abweichungen zwischen allen verfügbaren Daten und Informationen zum Gesundheitszustand und dazu passenden Pflegemaßnahmen durch KI und/oder durch zugehörige Regeln erkannt. Das neue Verfahren nutzt dabei nicht mehr nur die Daten und Informationen aus dem Pflegeprozess, sondern erkennt, wenn eine Pflegefachperson eine Pflegemaßnahme initiiert, die nicht bereits durch vorab dokumentierte Symptome des Patienten, die auch außerhalb der pflegerischen Einschätzung (Phase eins "reflexive Einschätzung") oder standardisierten Pflegemaßnahmen begründet ist. Die Künstliche Intelligenz (KI) oder regelbasierte Algorithmen analysieren den Kontext der von der Pflegefachperson initiierten Maßnahmen auf Abweichungen von typischen Mustern.

In einem zweiten Schritt der ereignisgesteuerten Navigation erfolgt eine Interaktion des elektronischen Dokumentationssystems mit der Pflegefachperson. Im Rahmen der Interaktion des elektronischen Dokumentationssystems mit der Pflegefachperson fragt das elektronische Dokumentationssystem die Pflegefachperson nach einer Begründung für die von der Pflegefachperson initiierten Maßnahmen.

Die Abfrage der Begründung kann durch eine intelligente Benutzeroberfläche erfolgen. Die intelligente Benutzeroberfläche schlägt eine Auswahl an möglichen Pflegediagnosen vor. Alternativ lässt die intelligente Benutzeroberfläche manuelle Eingaben der Pflegefachperson zu. Die intelligente Benutzeroberfläche kann vordefinierte Checklisten und/oder Freitextfelder zur Verfügung stellen.

In einem dritten Schritt erfolgt eine automatische Dokumentation in den einzelnen Phasen des Pflegeprozesses.

In dem dritten Schritt kann die Pflegefachperson Daten und/oder Informationen zur Unterstützung der zu treffenden Entscheidung abrufen.

In der Phase der ereignisgesteuerten Navigation können ergänzende Hinweise oder Vorschläge zu möglichen Diagnosen und/oder zur jeweils gewählten Maßnahmen des Pflegeprozesses gegeben werden. Das Verfahren unterstützt die Pflegefachperson auf diese Weise in deren Entscheidungsprozess.

In einem vierten Schritt erfolgt die Integration der durch die Pflegefachperson initiierten Phase des Pflegeprozesses in den gesamten klinischen Workflow. Die Dokumentation der Daten und/oder Informationen wird in Echtzeit aktualisiert. Interdisziplinäre Teams und andere Pflegefachpersonen erhalten zeitgleich Zugriff auf die Daten und/oder Informationen.

In diesem vierten Schritt können Verknüpfungen mit anderen Pflegesystemen hergestellt werden. Vorzugsweise, aber nicht ausschließlich, stellen die anderen Pflegesysteme Daten und/oder Informationen zu Laborwerten und/oder zu Vitalparametern und/oder Sensordaten bereit.

Mit Hilfe der Schritte der ereignisgesteuerten Navigation durch Hinzunahme von KI oder regelbasierter Algorithmen wird das intuitive Handeln der Pflegefachperson in Form von Daten und/oder Informationen systematisch erfasst. Das intuitive Handeln der Pflegefachperson wird in Form von Daten und/oder Informationen in den strukturierten, digitalen Workflow des Verfahrens integriert.

Die Schritte der ereignisgesteuerten Navigation ermöglichen eine agile Dokumentation der Daten und/oder Informationen, die den Pflegeprozess betreffen.

Die Schritte der ereignisgesteuerten Navigation verbessern die interdisziplinäre Kommunikation der Pflegekräfte unterschiedlicher medizinischer Fachgebiete. Die ereignisgesteuerte Navigation leistet somit einen bedeutenden Beitrag zur Qualitätssicherung in der Pflege.

Gleichzeitig wird sichergestellt, dass die pflegerische Expertise und die Erfahrungen der Pflegefachperson als wichtiger Bestandteil der Versorgung des Patienten in Form von Daten und/oder Informationen erhalten bleiben.

### Ausführungsformen des Verfahrens

Die Daten und/oder Informationen, die in dem elektronischen Dokumentationssystem des Eingabe-/Ausgabewerks abgespeichert sind, können bei dem Verfahren mit Hilfe eines Regelungssystems mit adaptiver Rückkopplung gesteuert werden.

Im Rahmen der ereignisgesteuerten Navigation können die Daten und/oder Informationen von der künstlichen Intelligenz (KI) auf Abweichungen gegenüber vorbestimmten Regeln und/oder Algorithmen überprüft werden.

Bei dem Verfahren interagiert die Pflegefachperson über eine intelligente Benutzeroberfläche mit dem elektronischen Dokumentationssystem.

Das Eingabe-/Ausgabewerk der Rechnereinheit kommuniziert zum Empfang und/oder zur Übermittlung von Daten und/oder Informationen mit wenigstens einem medizinischen Gerät.

### Vorteile der Erfindung

Das Verfahren betrifft einen nichtlinearen Pflegeprozess. Der nichtlineare Pflegeprozess umfasst iterative Phasen, die wiederholt und in gleicher oder ähnlicher Weise ablaufen können. Die Phasen des Pflegeprozesses verlaufen somit nicht strikt sequenziell, sondern iterativ und beeinflussen sich gegenseitig.

Die Erfindung stellt ein Verfahren zur Planung und/oder zur Modifikation und/oder zur Steuerung und/oder zur Bereitstellung von Daten und/oder Informationen für einen Pflegeprozess bereit, der flexibel, anpassungsfähig und nicht strikt sequenziell ist. Dabei berücksichtigt die Erfindung die Komplexität der Pflegesituation und steuert den Umfang der individuell notwendigen Dokumentation automatisch (⇒ Zeitersparnis).

Der Pflegeprozess wird den modernen Praxisanforderungen und neuesten wissenschaftlichen Erkenntnissen gerecht. Das Verfahren optimiert die Betreuung von Patienten mit komplexen Krankheitsbildern oder multiplen Problemen.

Die Pflegefachperson stellt zusätzlich eigenes, implizites Wissen zur Verfügung, mit dem die Pflegefachperson im Rahmen des Pflegeprozesses Entscheidungen treffen kann, die über das hinausgehen, was standardisierte Protokolle, Algorithmen und/oder Checklisten vorgeben könnten.

Das Verfahren berücksichtigt das implizite Wissen der Pflegekräfte, das in den herkömmlichen Pflegedokumentationssystemen nicht visualisiert wird. Das hatte bisher zur Folge, dass weitere am Behandlungsprozess Beteiligte, wie beispielhaft andere Pflegefachpersonen, Ärzte oder Physiotherapeuten beispielsweise über die Ursachen von verändertem Handeln nicht informiert waren und diese Erkenntnisse somit nicht für ihr eigenes Handeln berücksichtigen konnten. Das neue Verfahren dient also insbesondere der Verbesserung der Patientensicherheit.

Das implizite Wissen beschreibt Sachverhalte, die für die Pflegefachperson nicht aus sich selbst heraus zu verstehen sind. Die Pflegefachperson muss sich das implizite Wissen während des Pflegeprozesses vielmehr logisch erschließen.

Die nichtlineare Dokumentation von Daten und/oder Informationen zur Pflege des Patienten verbessert die Versorgung des Patienten.

Gleichzeitig erhöht sich die Nutzerfreundlichkeit der Rechnereinheit sowie weiterer elektronischer Systeme für die Pflegefachperson.

Mit Hilfe des neuen Verfahrens zur Planung und/oder zur Modifikation und/oder zur Steuerung und/oder zur Bereitstellung von Daten und/oder Informationen wird der bisherige lineare sequenzielle Pflegeprozess aufgebrochen. So wird die Pflegefachperson auch im komplexen klinischen Alltag bei fachlich oder kontextuell notwendiger Abweichung vom üblichen Dokumentationsprozess automatisiert angeleitet/ überwacht, um Fehler zu vermeiden und Risiken zu minimieren.

Mit Hilfe des Verfahrens wird eine fallbezogen sinnvolle Abfolge der einzelnen Phasen des Pflegeprozesses angeboten (ereignisgesteuerte Navigation). Dadurch verringert sich die Gefahr, dass die Pflegefachperson relevante Daten und/oder Informationen sowie Zusammenhänge nicht oder nur unzureichend berücksichtigt.

Das Verfahren zur Planung und/oder zur Modifikation und/oder zur Steuerung des Pflegeprozesses eines Patienten sowie zur Bereitstellung von Daten und/oder Informationen, bindet das intuitive Handeln der Pflegefachperson in den Prozess der digitalen Dokumentation der Daten und/oder der Informationen des Pflegeprozesses ein.

Der Wandel hin zur agilen Dokumentation mit bidirektionalen Phasen des Pflegeprozesses auf Basis eines Workflows im Rahmen des Verfahrens zur Planung und/oder zur Modifikation und/oder zur Steuerung eines Pflegeprozesses eines Patienten sowie zur Bereitstellung von Daten und/oder Informationen, stellt einen entscheidenden Fortschritt für die Dokumentation des Pflegeprozesses dar.

Der Wandel hin zur agilen Dokumentation mit bidirektionalen Phasen des Pflegeprozesses reduziert den bürokratischen Aufwand. Er verbessert die Arbeitsbedingungen der Pflegefachperson und er sorgt für eine höhere Versorgungsqualität der Patienten.

Die Gestaltung und die Dokumentation des Verfahrens unterstützt den Pflegealltag sinnvoll als praxisintegriertes Instrument zur Reflexion und Qualitätssicherung des Pflegeprozesses.

Interaktive Dashboards und Visualisierungen unterstützen die Pflegefachperson dabei, bestehende Phasen des Pflegeprozesses zu verstehen. Änderungen können schneller erfasst und Reaktionen sofort eingeleitet werden.

Zur Ausführung der ereignisgesteuerten Navigation steht eine modulare Dokumentationsansicht bereit.

Die Pflegefachperson kann mit Hilfe der modularen Dokumentationsansicht je nach Bedarf verschiedene Module individuell öffnen und/oder bearbeiten. Über die modulare Dokumentationsansicht kann die Pflegefachperson als Module eine Pflegeanamnese und/oder eine kontextuelle Syntheseseite und/ oder eine Pflegemaßnahmenplanung und/oder eine Pflegeevaluation und/oder einen Zwischenbericht individuell öffnen und bearbeiten. Die Module sind bidirektional miteinander verknüpft, so dass Änderungen in einem Modul automatisch auf relevante Bereiche eines anderen Moduls einwirken können.

Die Pflegefachperson kann den Pflegeprozess an die aktuellen Bedürfnisse des Patienten anpassen. Der Pflegeprozess kann dadurch flexibel und/oder individuell auf die jeweiligen Erfordernisse des Patienten angepasst werden.

Der Pflegeprozess kann iterativ und nichtlinear gestaltet werden. Die Pflegefachperson kann schneller und zielgerichteter auf Veränderungen des Gesundheitszustands des Patienten reagieren, so dass die Betreuung des Patienten wesentlich verbessert werden kann.

Zur Steuerung des Pflegeprozesses sieht das Verfahren wenigstens einen Rückkopplungsprozess vor. Eine kontinuierliche Rückkopplung und/oder eine iterative Anpassung der Maßnahmen des Pflegeprozesses verringern das Risiko von Fehlern und Missverständnissen im Pflegeprozess (Erhöhung der Patientensicherheit).

Die Pflegefachperson kann die zur Verfügung stehenden Ressourcen des Pflegeprozesses effizienter einsetzen. Der Pflegeprozess ist nicht starr auf bestimmte Phasen des Pflegeprozesses angewiesen. Die Pflegefachperson kann die Maßnahmen und/oder die Phasen des Pflegeprozesses in Echtzeit an die jeweiligen Gegebenheiten des Gesundheitszustands des Patienten anpassen.

Die Steuerung des Pflegeprozesses bewirkt eine effizientere Versorgung des Patienten.

Im Rahmen des Verfahrens zur Planung und/oder zur Modifikation und/oder zur Steuerung und/oder zur Bereitstellung von Daten und/oder Informationen, die einen Pflegeprozess eines Patienten betreffen, können KI-Modelle effektiv und situationsgerecht eingesetzt werden.

Rein beispielhaft, aber nicht ausschließlich, kann ein KI-Modell, basierend auf den Daten des Verfahrens, das Risiko eines Deliriums eines Patienten einschätzen. Eine präzise Steuerung der Phasen und/oder der Maßnahmen des Pflegeprozesses kann bei einem Patienten Delirien wirksam vermeiden.

Studien zufolge verursachen Delirien von Patienten im Gesundheitssystem zusätzliche Kosten von etwa 800 € bis 24.000 € pro Patient.

Neben den gesundheitlichen Vorteilen für den Patienten kann darüber hinaus die Notwendigkeit der Medikamenteneinnahme reduziert werden. Hierdurch können im Gesundheitssystem erhebliche Kosten eingespart werden.

Beispielhaft, aber nicht ausschließlich, kann das KI-Modell auch auf technische Vorrichtungen, wie z. B. Sensoren und/oder medizinische Geräte und/oder auf ein Bettensystem gerichtet sein.

### Zeichnungen

Weitere Beispiele und vorteilhafte Ausführungsformen der Erfindung werden nachfolgend anhand der Figuren näher beschrieben. Hierbei zeigen:
Fig. 1, ein Beispiel ereignisgesteuerter Navigation,
Fig. 2, in schematischer Darstellung das Regelwerk des Pflegeprozesses, und
Fig. 3, die Aufbereitung der Daten und/oder Informationen des Pflegeprozesses.

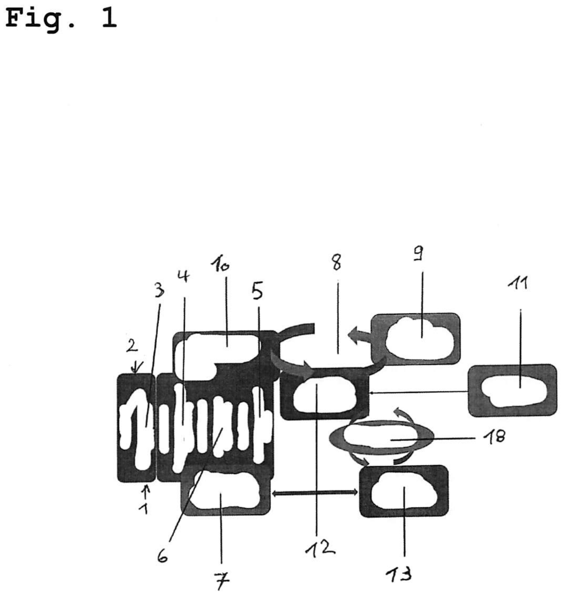

Fig. 1 zeigt ein Beispiel für eine ereignisgesteuerte Navigation.

Quer zur Darstellung der Fig. 1 ist im Bereich 1 das implizite Wissen der Pflegefachperson gezeigt, wogegen mit der Bezugsziffer 2 das explizite Wissen bzw. die expliziten Kenntnisse der Pflegefachperson dargestellt sind.

Die Daten und/oder Informationen des Pflegeprozesses sind im Bereich der Bezugsziffer 3 geclustert dargestellt.

Die Bezugsziffer 4 zeigt, dass diagnostische Hypothesen gebildet werden.

Bei Bezugsziffer 6 werden die Hypothesen vorzugsweise, aber nicht ausschließlich, mit Hilfe der KI überprüft.

Auf Grundlage des expliziten Wissens der Pflegefachperson werden bei der Bezugsziffer 5 Pflegediagnosen gebildet und bei Bezugsziffer 10 kontextualisierte Wissensintegration durch die Synthesebildung visualisiert.

Auf Grundlage ihres impliziten Wissens kann die Pflegefachperson bei Bezugsziffer 7 in den Pflegeprozess eingreifen.

Die Sammlung der Daten und/oder Informationen, die den Pflegeprozess betreffen, erfolgt bei Bezugsziffer 8.

Die reflexive Einschätzung der Phasen des Pflegeprozesses durch die Pflegefachperson erfolgt bei Bezugsziffer 12.

Externe Datenquellen für den Bezug von Daten und/oder Informationen sind mit Bezugsziffer 9 gezeigt.

Die Daten und/oder Informationen zur Gesundheitsgeschichte des Patienten sind durch Bezugsziffer 11 dargestellt.

Die ereignisgesteuerte Navigation an sich ist im Bereich der Bezugsziffer 18 gezeigt.

Adaptive Maßnahmen im Pflegeprozess, die von der Pflegefachperson initiiert werden, sind mit Bezugsziffer 13 angegeben.

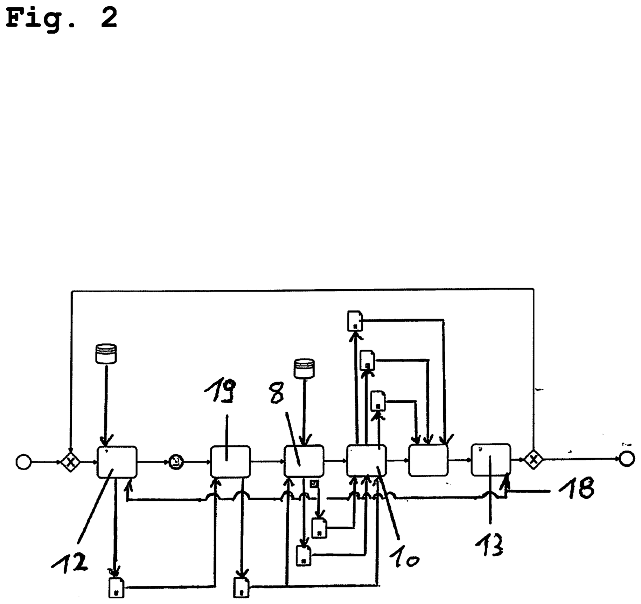

Fig. 2 zeigt in schematischer Darstellung das Regelwerk des Pflegeprozesses.

Mit Bezugsziffer 12 ist die reflexive Einschätzung dargestellt.

Die Ableitung etwaiger Gesundheitsrisiken findet bei Bezugsziffer 19 statt.

Die Daten und/oder Informationen sind bei Bezugsziffer 8 dargestellt.

Bei Bezugsziffer 10 erfolgt die kontextualisierte Wissensintegration durch die Synthesebildung.

Bei Bezugsziffer 13 wird in Fig. 2 die adaptive Maßnahmenplanung dargestellt.

In der Fig. 2 wird die ereignisgesteuerte Navigation durch die Bezugsziffer 18 dargestellt.

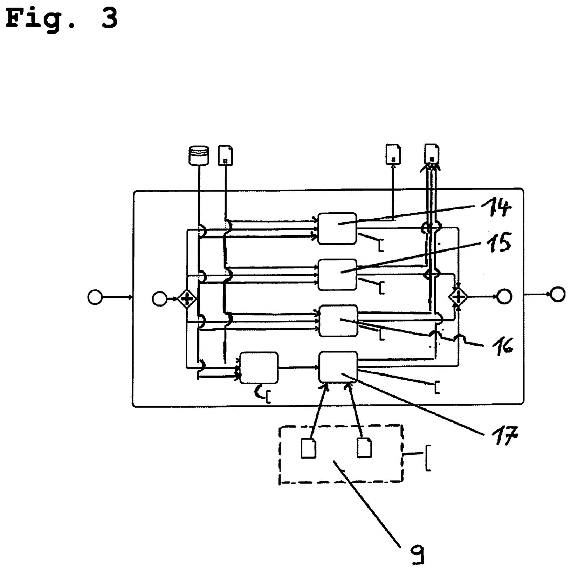

In Fig. 3 ist die Aufbereitung der Daten und/oder Informationen des Pflegeprozesses gezeigt.

Bei der Bezugsziffer 14 der Fig. 3 werden die Ressourcen bestimmt, die dem Pflegeprozess zur Verfügung stehen.

Bei den Bezugsziffern 15-17 werden die abgeleiteten Pflegediagnosen (geclustert nach Fokus-, Basis- und Risikodiagnose) dargestellt.

Die Sammlung und Visualisierung der externen Daten und/oder Informationen des Pflegeprozesses erfolgt bei Bezugsziffer 9 der Fig. 3

### Bezugsziffernliste

- 1: Implizites Wissen
- 2: Explizites Wissen
- 3: Clusterung
- 4: Diagnostische Hypothesen
- 5: Pflegediagnosen
- 6: Überprüfung der Hypothesen
- 7: Intervention im Pflegeprozess
- 8: Sammlung der Daten und/oder Informationen
- 9: Externe Datenquellen
- 10: Kontextualisierte Wissensintegration (Synthesebildung)
- 11: Gesundheitsgeschichte des Patienten
- 12: Reflexive Einschätzung
- 13: Adaptive Maßnahmen
- 14: Ressourcen zur Pflegemaßnahme
- 15: Fokusdiagnosen bestimmen
- 16: Basisdiagnosen bestimmen
- 17: Risikodiagnosen bestimmen
- 18: Ereignisgesteuerte Navigation (Loop-Funktion)
- 19: Risikoauswertung

## Patentansprüche

1. Verfahren zur Planung und/oder zur Modifikation und/oder zur Steuerung und/oder zur Bereitstellung von Daten und/oder Informationen, die die pflegerische Versorgung im Pflegeprozess eines Patienten betreffen,
- wobei die Daten und/oder Informationen in einem elektronischen Dokumentationssystem eines Eingabe-/Ausgabewerks einer Rechnereinheit abgespeichert werden, und
- wobei die Daten und/oder Informationen zur weiteren Verarbeitung vom Eingabe-/Ausgabewerk wenigstens einer weiteren Komponente der Rechnereinheit zugeführt werden,
**dadurch gekennzeichnet, dass**
- die Planung und/oder die Modifikation und/oder die Steuerung und/oder die Bereitstellung der Daten und/oder Informationen auf Basis einer ereignisgesteuerten Navigation beruht,
- wobei die Daten und/oder Informationen im Rahmen der ereignisgesteuerten Navigation der Analyse durch künstliche Intelligenz (KI) unterliegen, und
- die Daten und/oder Informationen auf einer Interaktion des elektronischen Dokumentationssystems mit wenigstens einer Pflegefachperson beruhen.

2. Verfahren nach Anspruch 1, **dadurch gekennzeichnet, dass** die Daten und/oder Informationen, die in dem elektronischen Dokumentationssystem des Eingabe-/Ausgabewerks abgespeichert sind, in einem Regelungssystem mit adaptiver Rückkopplung gesteuert und/oder geregelt werden.

3. Verfahren nach Anspruch 1, **dadurch gekennzeichnet, dass** die künstliche Intelligenz (KI) im Rahmen der ereignisgesteuerten Navigation die Daten und/oder Informationen auf Abweichungen gegenüber vorbestimmten Regeln und Algorithmen überprüft.

4. Verfahren nach Anspruch 1, **dadurch gekennzeichnet, dass** das elektronische Dokumentationssystem über eine intelligente Benutzeroberfläche mit der Pflegefachperson interagiert.

5. Verfahren nach Anspruch 1, **dadurch gekennzeichnet, dass** das elektronische Dokumentationssystem mit externen Pflegesystemen kommuniziert.

6. Verfahren nach Anspruch 1, **dadurch gekennzeichnet, dass** das Eingabe-/Ausgabewerk der Rechnereinheit zum Empfang und/oder zur Übermittlung von Daten und/oder Informationen mit wenigstens einem medizinischen Gerät kommuniziert.
